# EUROPEAN PATENT SPECIFICATION

(11) **EP 4 412 533 B1**
(45) Date of publication and mention of the grant of the patent: **28.10.2026**
(21) Application number: 22789287.4
(22) Date of filing: 06.10.2022
(51) Int. Cl.: A61B 10/02, A61B 1/303, A61M 29/02, A61B 1/00, A61B 10/00

(54) **CERVICAL INSPECTION DEVICE, METHODS OF CONTROL THEREOF, AND CERVICAL SAMPLING BRUSH**
VORRICHTUNG ZUR ZERVIKALEN INSPEKTION, VERFAHREN ZUR STEUERUNG DAVON UND BÜRSTE ZUR ZERVIKALEN PROBENAHME
DISPOSITIF D'INSPECTION CERVICALE, SES PROCÉDÉS DE COMMANDE ET BROSSE D'ÉCHANTILLONNAGE CERVICAL

(30) Priority: 07.10.2021 GB 202114384
(43) Date of publication of application: 14.08.2024
(73) Proprietor: LBN INNOVATIONS LTD, Ealing Greater London W5 1EN (GB)
(72) Inventor: BEN-NATHAN, Louise Emma, Ealing Greater London W5 1EN (GB); BLONDECK, Alex, Usk Gwent NP15 1SY (GB); BADHAM, Gary, Usk Gwent NP15 1SY (GB); HELMICH, Mark, Usk Gwent NP15 1SY (GB)
(74) Representative: Doherty, William
(86) International application number: PCT/GB2022/052521
(87) International publication number: WO 2023/057755

(56) References cited:
- WO-A1-2017/124755
- WO-A2-2020/096666
- US-A1- 2002 165 467
- US-A1- 2008 188 769
- US-A1- 2017 303 903
- US-A1- 2021 137 506

## Description

The present invention relates to a cervical inspection device for facilitation of a cervical smear test. The invention also relates to a method of controlling a cervical inspection device for performing a cervical smear test. The invention further relates to a cervical sampling brush particularly but not exclusively for use in performing cervical smear tests.

Every woman over the age of twenty-five and a half is invited to a cervical smear screening test every three to five years. The intention is to collect a sample from the transformation zone of the cervix, which contains the squamocolumnar junction. This test collects cells from the transformation zone of the cervix to look for pre-cancerous changes. However, the transformation zone cannot be seen by the human eye, and the squamocolumnar junction changes position with age. To obtain these cells a speculum is currently placed into the vagina and opened, which then allows the medical practitioner to visualise the cervix through the centre of the speculum. Once the cervix is visualised a collection device is inserted through the opened speculum and brushed over the cervix. The cells on the brush are then analysed for human papillomavirus (HPV) which if found is then analysed further for indications of cancer.

The brush commonly used in the UK is referred to as a Rovers (RTM) brush, which has a shaped head with flexible bristles adapted to the contours of the transformation zone of the cervix. This is an improvement over rigid spatulas and other less considerate endocervical devices. Rigid spatulas are also not compatible with the cytology solution used in the NHS in the UK. However, this brush is difficult to extract without loss of sample on extraction from the vagina, since touching the vaginal walls causes cross-contamination and potential loss of sample. In 2020, 40,000 samples were deemed inadequate. Lubrication can also cause contamination of samples, as can vaginal bleeding.

Furthermore, 1 in 4 women in the UK do not attend their cervical screenings. In 2020 this amounted to approximately 1.4 million women; in 2021 this had increased to 1.56 million. Embarrassment on body shape, pubic hair and shape of their vulva are considered to be contributing factors. Inability to get an appointment at a convenient time is another. Pain is also postulated as being a contributing factor and this is not readily acknowledged for fear it will put off women turning up for the test. When women do express pain and are unable to complete the test, they are often made to feel that they are the problem, rather than the discomfort of the test itself. However, this has significant consequences; it is estimated that 850 women die every year from cervical cancer, with a great percentage of these deaths being from non-white minority ethnic groups. Globally, the World Health Organization anticipates an annual increase in the number of new cases of cervical cancer to increase from 570,000 to 700,000 between 2018 and 2030, with anneal deaths increasing from 311,000 to 400,000. Improved testing rates would help to reverse this trend.

Discomfort can be increased if a woman has a retroverted uterus, which occurs in around 1 in 5 patients. This means that instead of the uterus sitting forward over the bladder, it is directed backwards, or an alternative unorthodox position, over the rectum. If a woman has a retroverted uterus, when the smear test is performed whilst the patient is lain on her back, the cervix will not be linearly aligned to the vaginal canal. As such, a greater degree of force and movement of the speculum is required, which can cause pain, discomfort, and damage. The inability to use lubrication also increases the discomfort to the patient. Nulliparity, vaginismus, atrophy, and prior trauma are also possible reasons for painful examination, in addition to the selection of the incorrect size of speculum.

Examples of cervical sampling devices known in the art are provided in US2021/137506A1, US2008/188768A1, US2002/165467A1, US2017/303903A1, WO2017/124755A1, and WO2020/096666.

The present invention seeks to provide a means of performing a smear test accurately without the use of a speculum, as well as improving the ease with which the cervix can be located for the test.

According to the invention, there is provided a cervical inspection device for performing a cervical smear test, the device comprising: a tubular body having a first end which is engagable by an operator and a second end which is insertable into a patient's vaginal canal, the tubular body having a central bore which extends along its longitudinal axis for receiving a cervical sampling brush therethrough; an expansion element at to the second end of the tubular body; and control means operable from the first end of the tubular body for selectably expanding and/or contracting the expansion element; and the expansion element being expandable and/or contractable at the second end of the tubular body without altering a dimension of the tubular body, the expansion element forming a circumferentially-bound protective region beyond the central bore in an expanded condition thereof for the said cervical sampling brush to move through during a cervical smear test; and characterised by a camera at or adjacent to the second end of the tubular body and positioned radially outwardly of and spaced apart from the central bore and radially inwardly of the expansion element; and the expansion element comprises a flexible hood, the control means comprising a plurality of tabs for splaying the flexible hood into an open condition, the plurality of tabs each being connected to the second end of the tubular body by a hinge.

The provision of a cervical inspection device capable of protecting the path for the cervical sampling brush thereof all the way to the transformation zone of the cervix ensures that there is no cross-contamination of the sample. Providing an expansion element and mechanism for control thereof allows for the walls of the vaginal canal to be gently urged out of the way, ensuring that a good visual path is provided between the camera and the transformation zone, and also providing a suitable access route for the cervical sampling brush. It will also be appreciated that, since there is a central bore in the tubular body, this could also be utilised for other processes, such as introducing a sprayer device to spray the cervix with acetic acid, since this permits identification of the location of the transformation zone. Similarly, colposcopic tools could also be introduced in this manner.

The tubular body may comprise a brush housing which includes a channel forming the central bore.

If a dedicated unit is provided which forms the channel, this can be formed so as to be a housing for the cervical sampling brush once the sample is taken. This can further prevent contamination of the sample.

The brush housing may preferably be removable from the tubular body.

If the brush housing is completely removable, then the cervical sampling brush need never be exposed during the transfer process from sampling to cytology laboratory. This greatly reduces the risk of sample failure in transit.

Additionally, or alternatively, the tubular body may be a two-part body having a leading portion which is removable from a trailing portion.

In such an arrangement, the leading portion of the tubular body could be formed to be disposable, so that an operator of the device can swap between leading portions between patients, to minimise the risk of cross-contamination.

Preferably, there may be a camera receiving area at or adjacent to an end of the channel which is laterally spaced apart from the central bore and within a maximum lateral extent of the expansion element in the expanded condition. The camera receiving area may preferably have a window.

The location of a camera for the device is important. The point of any camera is to validate whether the tip of the brush enters the cervical opening, known as the internal cervical os, whilst the brush is in situ. As such, the camera needs to be present at the same time as the cervical sampling brush, but also not occluded by the protection region formed by the expansion element. The window of the brush housing provides a suitable barrier to prevent contamination of the camera lens, allowing the more complex parts of the cervical inspection device to be protected from the patient. This improves the between-patient sanitization process.

Having an in-built camera with the cervical inspection device greatly increases the utility of the device as a whole.

Preferably, the camera may be angled towards the central bore for monitoring the cervical sampling brush in use. There may also be an illumination element associated with the camera.

It is preferred that the camera be directed towards the expected target of the cervical sampling brush, to improve the accuracy of positioning of the cervical inspection device by the operator. Illumination can also assist with the identification of the correct cervical area.

Optionally, the expansion element may have a conical, frusto-conical, substantially conical, or substantially frusto-conical shape in the expanded condition.

A protection zone shaped in this manner provides ample room for manoeuvre of the cervical sampling brush, to ensure that the sample is extracted safely specifically from the transformation zone of the cervix.

A distal rim of the expansion element may in-use form a cervical-contact edge of the protection zone to form an enclosure with the cervix.

The expansion element could make direct physical contact with the cervix to thereby create a sealed region within which the cervical sampling brush can move without risk of contamination from, for example, the vaginal walls.

The cervical inspection device may further comprise a sheath which is receivable over the tubular body.

A sheath overlying the tubular body provides a suitably santizable and/or disposable outer layer for the cervical inspection device which can be replaced or cleaned following use. This protects the moving and delicate parts of the tubular body.

Preferably, the sheath may be connected to the brush housing.

If the sheath and brush housing are connected with one another, they can be removed as a unit, with the sheath providing additional protection for the sample enclosed within the brush housing on removal.

Optionally, an end of the sheath may form the flexible hood.

The outer layer of the sheath, being flexible, provides a suitable material for use as the flexible hood of the engagement element.

Preferably, the sheath may be formed from a silicone material.

Silicone is wipe-clean and easily disinfected, as well as providing a smooth outer surface for the cervical inspection device which will not cause pain to the patient on insertion.

In one embodiment, the control means may comprise a moveable member of the tubular body.

A moveable part of the tubular body itself, such as a sliding or driven cylinder, may be one simple mechanical means of providing a remote force at the second end of the cervical inspection device, suitable for controlling the expansion and contraction condition of the expansion element.

Preferably, the control means may engage with the expansion element in an interference fit to urge the expansion element into the expanded condition.

Wedging engagement is a natural means of creating radial expansion, which is desirable for the expansion characteristics of the expansion element.

The cervical inspection device preferably further comprises a cervical sampling brush comprising: an elongate stem; and a cervical sampling head having a central hub and a lateral support, the cervical sampling head having a plurality of flexible sampling bristles thereon; wherein in a sampling condition, the lateral support has a first lateral extent relative to the elongate stem, and in a retraction condition, the lateral support is movable or foldable towards an axis of the elongate stem to adopt a second lateral extent which is less than the first lateral extent, so as to be receivable into the tubular body without the plurality of flexible sampling bristles contacting the tubular body or the expansion element.

Optionally, the lateral support of the cervical sampling brush may be engaged to the central hub via a hinge member.

Preferably, the hinge member may be a resiliently flexible hinge.

Possible arrangements of the hinge member include: where a hinge axis for the lateral support is parallel or substantially parallel with a longitudinal axis of the elongate stem; or wherein a pair of diametrically opposed lateral supports is provided, respectively connected to the central hub via opposed said hinge members, wherein hinge axes of the hinge members are parallel to one another; or wherein a pair of diametrically opposed lateral supports is provided, respectively connected to the central hub via opposed said hinge members, wherein hinge axes of the hinge members are co-axial.

A pair of diametrically opposed lateral supports may be provided respectively connected to the central hub via opposed said hinge members, the lateral supports having rotationally symmetric arcuate shapes.

The lateral supports may be formed as linear or fan-shaped supports.

The or each lateral support may have a tapered bristle-distal edge to urge the or each lateral support into the retracted condition upon application of an axial force.

Optionally, the central bore may be at least in part dimensioned to receive the cervical sampling brush therethrough when the cervical sampling head is in the retracted condition.

The central bore of the cervical inspection device may be designed so that it naturally urges the cervical sampling brush into the retracted condition, eliminating the need for complex mechanisms for control of the sampling brush retraction conditions.

According to a further aspect of the invention, there is provided a method of controlling a cervical inspection device for performing a cervical smear test, the method comprising the steps of: providing a cervical inspection device in accordance with the second aspect of the invention, the expansion element being in a contracted condition; and using the control means, expanding the expansion element into the expanded condition to a protective region beyond the central bore through which a cervical sampling brush can pass.

According to another aspect of the invention, there is provided a method of controlling a cervical inspection device for performing a cervical smear test, the method comprising the steps of: providing a cervical inspection device in accordance with the second aspect of the invention; pushing the cervical sampling brush through the central bore of the tubular body to permit opening of the cervical sampling head into the sampling condition; and pulling the cervical sampling brush into the central bore of the tubular body to urge the lateral supports of the cervical sampling brush to fold towards the central hub upon contact of the lateral supports with the tubular body.

Automatic closure of the cervical sampling brush on retraction provides a simple means of using a cervical inspection device which obviates the need for a speculum, without needing to prepare complex moving parts.

The invention will now be more particularly described, by way of example only, with reference to the accompanying drawings, in which:
Figure 1 shows a perspective representation of a first embodiment of a cervical inspection device in accordance with the the invention for performing a cervical smear test;
Figure 2 shows a perspective representation of the cervical inspection device of Figure 1 in an inspection condition;
Figure 3 shows an exploded perspective representation of the cervical inspection device of Figure 1;
Figure 4 shows a perspective representation of a second embodiment of a cervical inspection device in accordance with the invention for performing a cervical smear test, the device including a cervical sampling brush ;
Figure 5A shows a perspective representation of the device body of the cervical inspection device of Figure 4 in a retracted condition;
Figure 5B shows the device body of Figure 5A in an inspection condition;
Figure 6 shows an end view of the cervical inspection device of Figure 4;
Figure 7 shows a perspective representation of a first embodiment of a cervical sampling brush , in a closed condition;
Figure 8 shows a plan view of the cervical sampling brush of Figure 7;
Figure 9 shows a perspective representation of the cervical sampling brush of Figure 7, in a sampling condition;
Figure 10 shows a plan view of the cervical sampling brush of Figure 9;
Figure 11A shows an end view of the cervical sampling brush of Figure 7;
Figure 11B shows a side view of the cervical sampling brush of Figure 11A;
Figure 11C shows an end view of the cervical sampling brush in Figure 9;
Figure 11D shows a side view of the cervical sampling brush of Figure 11C;
Figure 12A shows a perspective representation of a cervical sampling head of the cervical sampling brush of Figure 7, interacting with a brush housing of a cervical inspection device, in a retracted state;
Figure 12B shows a perspective representation of the cervical sampling head and brush housing of Figure 12A, in an extended state;
Figure 13A shows a plan view of a second embodiment of cervical sampling brush , in an extended state;
Figure 13B shows a perspective view of the cervical sampling brush of Figure 13A;
Figure 13C shows an end view of the cervical sampling brush of Figure 13A;
Figure 13D shows a side view of the cervical sampling brush of Figure 13A;
Figure 14A shows a plan view of the cervical sampling brush of Figure 13A, in a retracted state;
Figure 14B shows a perspective view of the cervical sampling brush of Figure 14A;
Figure 14C shows an end view of the cervical sampling brush of Figure 14A;
Figure 14D shows a side view of the cervical sampling brush of Figure 14A;
Figure 15A shows a plan view of a third embodiment of cervical sampling brush , in an extended state;
Figure 15B shows a perspective view of the cervical sampling brush of Figure 15A;
Figure 15C shows an end view of the cervical sampling brush of Figure 15A;
Figure 15D shows a side view of the cervical sampling brush of Figure 15A;
Figure 16A shows a plan view of the cervical sampling brush of Figure 15A, in a retracted state;
Figure 16B shows a perspective view of the cervical sampling brush of Figure 16A;
Figure 16C shows an end view of the cervical sampling brush of Figure 16A;
Figure 16D shows a side view of the cervical sampling brush of Figure 16A;
Figure 17A shows a plan view of a fourth embodiment of cervical sampling brush, in a retracted state;
Figure 17B shows a perspective view of the cervical sampling brush of Figure 17A;
Figure 17C shows an end view of the cervical sampling brush of Figure 17A;
Figure 17D shows a side view of the cervical sampling brush of Figure 17A;
Figure 18A shows a plan view of the cervical sampling brush of Figure 17A, in an extended state;
Figure 18B shows a perspective view of the cervical sampling brush of Figure 18A;
Figure 18C shows an end view of the cervical sampling brush of Figure 18A;
Figure 18D shows a side view of the cervical sampling brush of Figure 18A;
Figure 19A shows a front perspective representation of a further embodiment of a cervical inspection device in accordance with the invention for performing a cervical smear test, in a retracted condition;
Figure 19B shows a front perspective representation of the cervical inspection device of Figure 19A, in an expanded condition;
Figure 20A shows a longitudinal cross-sectional representation of the cervical inspection device of Figure 19A; and
Figure 20B shows a longitudinal cross-sectional representation of the cervical inspection device of Figure 19B.

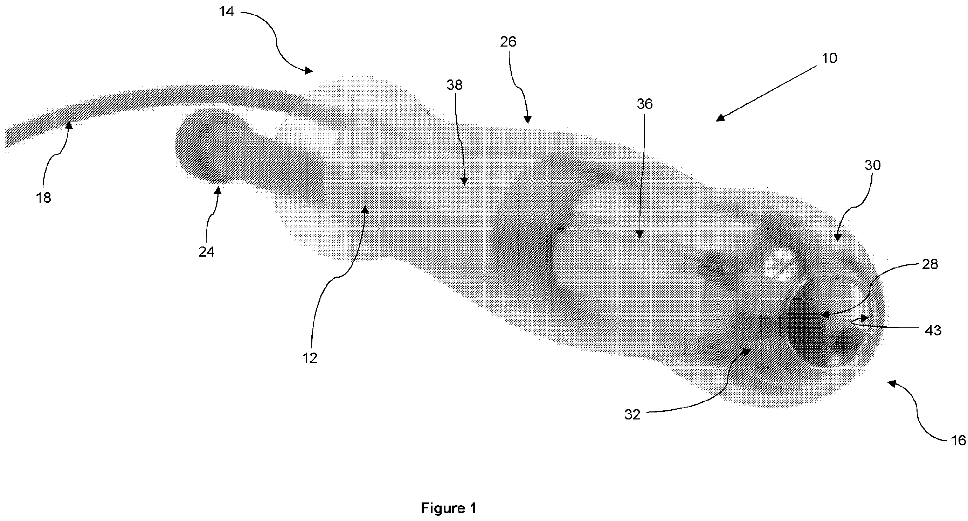

Referring to Figure 1, there is indicated a cervical inspection device, referenced globally at 10, which is adapted for performing a cervical smear test, in a manner which also permits inspection of the transformation zone of the cervix in use.

The cervical inspection device 10 has a tubular body 12 which has a first end 14 which is engagable by an operator, typically a nurse, and a second end 16 which is insertable into the patient's vaginal canal. A power cable 18 can be seen extending through the first end 14 to provide a power supply to the electronic components of the apparatus. It will be apparent that the power cable 18 could also be used as the conduit for information transfer to and from the device 10, such as a video feed, and that this could additionally or alternatively be performed wirelessly.

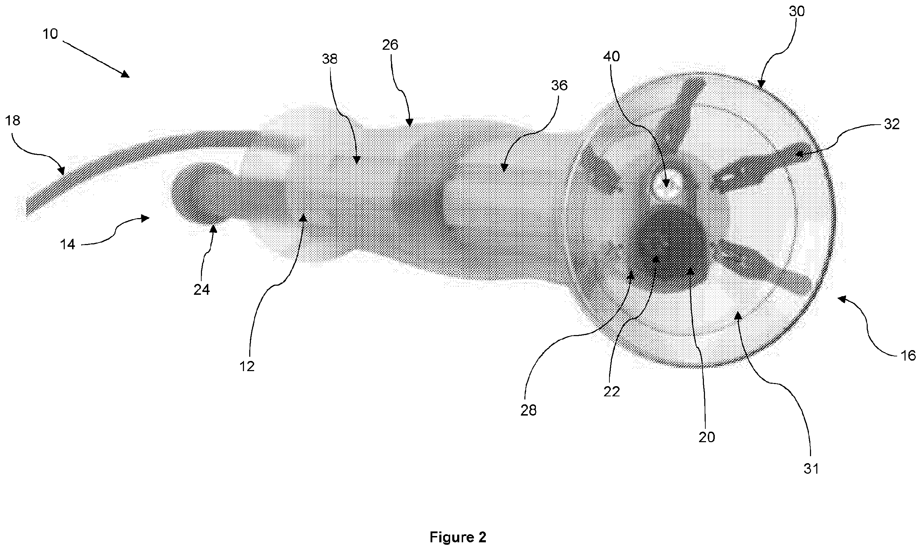

The tubular body 12 has a central bore 20 which extends along a longitudinal axis thereof, and this is best seen in Figure 2. A cervical sampling brush 22 can be seen housed inside the central bore 20 in a retracted state in Figure 2, the brush 22 being linearly advanced using a manually operable handle 24. The central bore 20 is designed to be dimensioned to receive the cervical sampling brush 22 therein when the cervical sampling brush 22 is in a retracted state. This will be discussed in more detail below.

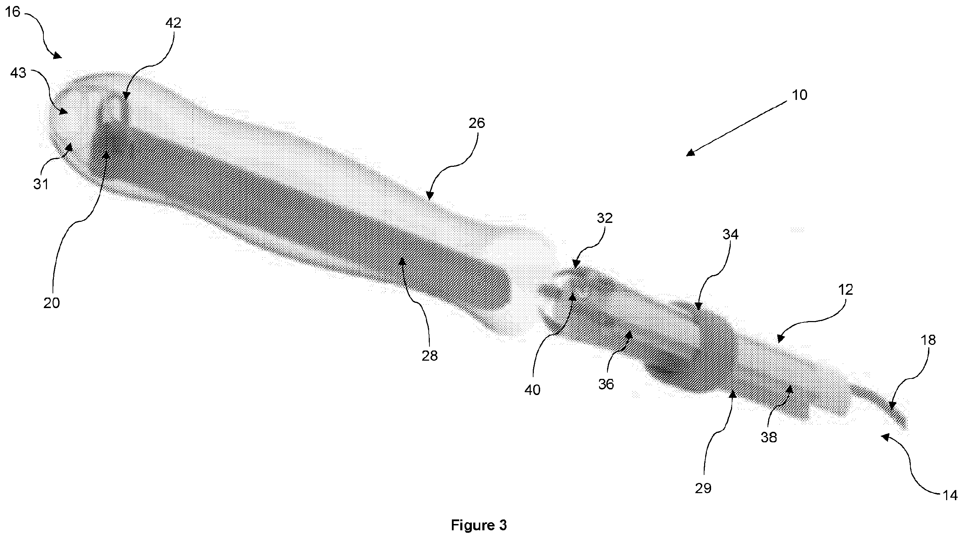

The cervical inspection device 10 is here provided with a sheath 26 receivable over the tubular body 12. This provides a smooth insertion surface to prevent snagging on the walls of the vaginal canal. The sheath 26 may preferably be formed from a silicone material, or similarly disinfectable, sterilisable, or wipe-clean material. Disposable materials could also be considered, such as a paper sheath. The sheath 26 is shown removed from the tubular body 12 in Figure 3. It will be apparent, however, that a sheath could be dispensed with.

The sheath 26 may be preferably connected to a, preferably disposable, brush housing 28 receivable into the tubular body 12 which forms the central bore 20. Alternatively, the main tubular body could form the brush housing directly. The brush housing 28, which may be a rigid unit, provides a physical barrier between the patient and the tubular body 12, which prevents contamination of the cervical inspection device 10 between patients. The brush housing 28 may therefore slot into a corresponding channel 29 formed in the tubular housing 12. The sheath 26 and brush housing 28 thus together form a complete barrier layer for the tubular housing 12, thereby allowing it to be inserted into a patient's vaginal canal without risk of contamination. The sheath 26 and brush housing 28 thus may be provided as a disposable unit, comprising external and internal barriers for the tubular housing 12 respectively.

This brush housing 28 can also act as a transferrable housing for the cervical sampling brush 22 to a cytological analysis laboratory, where this process is used, reducing the risk of cross-contamination. It will be apparent that the brush housing 28, where provided, need not necessarily be connected to the sheath 26; this is merely an advantageous feature which allows to two components to be safely removed from the tubular body 12 as one for sample transfer. In the depicted embodiment, however, the sheath 26 covers the end of the tubular housing 12 not otherwise covered by the brush housing 28, thereby completing the barrier between the patient and the tubular housing 12. This configuration allows the sheath 26 to have an open end through which the brush 22 can penetrate.

It is preferred that the cervical inspection device 10 include an expansion element 30 which is openable to allow for improved inspection capability and protection at the transformation zone of the cervix, since such an expansion element 30 can push the walls of the vaginal canal out for improved visuals and to allow the brush to unfurl in an unhindered manner. The expansion element 30 also forms a protection region around the transformation zone, preferably but not necessarily engaging with the cervix directly in use at a cervix-contact edge thereof, so that the brush can be safely advanced therethrough so that it only contacts the transformation zone and is not contaminated, for example, by the vaginal walls of the patient. The expansion element 30 could of course stop short of contacting the cervix, whilst still also creating or substantially creating a protection region.

In the illustrated embodiment of Figures 1 to 3, the expansion element 30 is provided as a flexible hood 31 of the sheath 26, operated by a control means. The control means comprises a plurality of tabs 32 which are arranged to urge the expansion element 30 between the closed and open positions respectively shown in Figures 1 and 2. The flexible hood 31 covers the inside and outside surfaces of the tabs 32, so that they are occluded from the vaginal canal in use. The flexible hood 31 can thus connect to the outer edge of the brush housing 28 in order to form a continuous barrier protecting the tubular housing 28.

The tabs 32 are hingeably connected to the second end 16 of the tubular body 12, being pivotable by a linear slider 34 which is positioned on the outside of the tubular body 12, typically under the sheath 26. Pushing the slider 34 towards the second end 16 will effectively push the tabs 32 into the closed condition based on the relative connection points of the pivot points with respect to a wire connection 36 between the slider 34 and the tabs 32. Pulling the slider 34 pulls on the wire connections 36 pulling the tabs 32 outwards, and opening the expansion element 30. The tubular body 12 may optionally have runners 38 within which the wire connections 36 are moveable, extending parallel to the longitudinal axis.

Whilst the sheath 26 in the depicted embodiment provides the flexible hood of the expansion element 30, it will be apparent that the expansion element could be formed as a unitary component, without the need to provide a separate sheath. The expansion element would thereby form the protection region by itself.

Whilst a slider-and-wire arrangement is shown, there could equally be provided a moveable member within the tubular body 12 which controls the expansion and/or contraction function of the expansion element 30. For example, a movable cylinder within the tubular body could be provided, which at least in part forms the central bore, and a distal end of such a cylinder could be formed so as to create an interference fit with the base of the expansion element. This cylinder would then urge against the tabs of the expansion element, to splay them outward and create a protection region. Such tabs are hinged to the tubular body. Advantageously, since the splaying of the expansion element 30 occurs at or adjacent to the second end 16 of the cervical inspection device 10, there is no increase in dimension of the tubular body 12 within the vagina, which would otherwise cause pain to the patient.

The use of the expansion element 30 to improve visual access to the transformation zone is useful since the cervical inspection device 10 includes a camera 40 at or adjacent to the second end 16. This may be protected via a camera receiving area, here illustrated as a camera window 42, which may be formed as a transparent or translucent cover on the brush housing 28. Preferably, there will also be an illumination element, such as an LED, associated with the camera 40 to ensure that visual inspection is feasible. A video feed may be transmitted via the same cable as the power cable 18. An aperture 43 forming the open end of the sheath 26 allows for the camera 40 to have a viewing field including the cervix on entry, in addition to providing access for the brush 22. The aperture 43 provides an unobscured visual path for the camera 40 even when the expansion element 30 is in the closed condition.

The camera 40 is positioned laterally or radially outwardly of the central bore 20, so as not to obstruct the cervical sampling brush 22 in use. To this end, the camera 40 is preferably angled towards the central bore 20 so as to be able to monitor the movements of the cervical sampling brush 22 in use. Crucially, the camera 40 is positioned in such a manner as to be able to inspect inside the protection region, that is, the region created about the cervix in-use by the expansion element 30. Whilst the camera 40 is thus spaced apart from the central bore 20, it does remain within the lateral or radial extent of the distal end of the expansion element 30, when in the expanded condition.

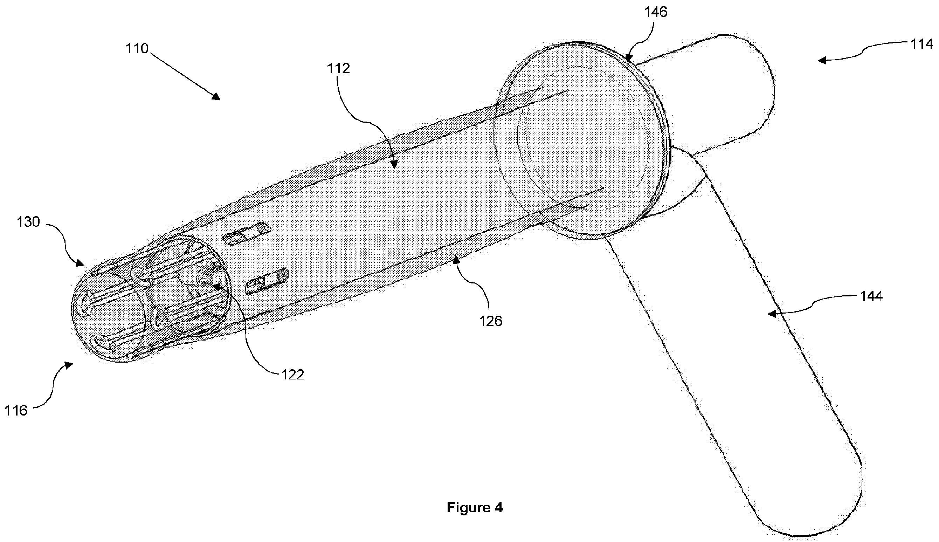

An alternative embodiment of cervical inspection device 110 is shown in Figure 4, embodying many of the same principles as the first embodiment. As such, identical or similar features of the first embodiment will be referenced using identical or similar reference numerals, and further detailed description will be omitted for brevity.

The cervical inspection device 110 is shown with an extended handle 144, which allows the user greater control over the whole device 110. A flange 146 is provided towards the first end 114 of the tubular body 112 in order to improve grip. It is not intended as a means of identifying an insertion depth of the device 110, since the device 110 is usable for all patients, which is a significant improvement over speculums having different dimensions.

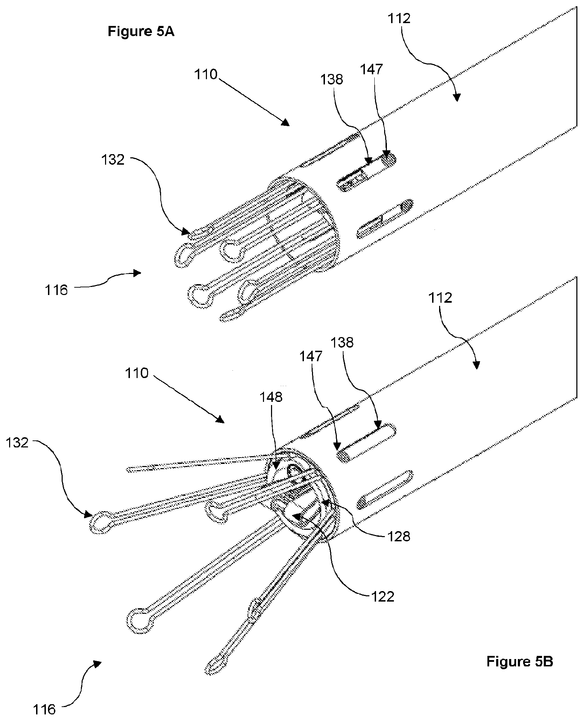

The external slider is absent from the indicated embodiment; instead, the tabs 132, which are here formed as bent wire formations, are operated via movement of the tubular housing 112 itself. The range of movement of the tabs 132 can be seen in detail in Figures 5A and 5B. The brush housing 128 of the cervical inspection device 110 is adapted to be fixed relative to the tabs 132. The tabs 132 are provided so as to be outwardly biased, for example, by a spring member, which urges them into the open condition. When the tubular housing 112 is in a forward position towards the second end 116 of the device, as shown in Figure 5A, pins 147 are held captive towards a rear end of runners 138. The tabs 132 are held in position within the end of the tubular body 112 by compression of the tabs 132 towards one another by the internal surface of the tubular body 112. As the tubular body 112 is urged backwards, the pins 147 become positioned relatively forward within runners 138, until a spring force of the tabs 132 overcomes the compression thereof, and the tabs 132 become splayed relative to one another.

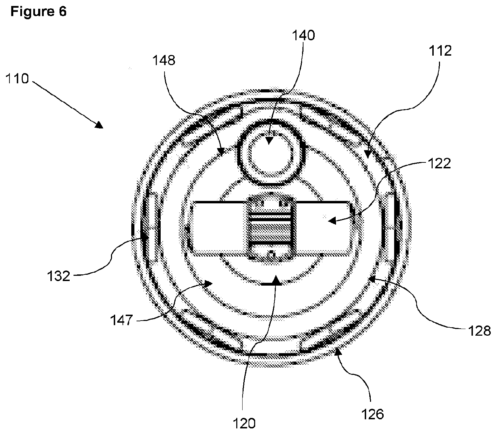

Figure 6 shows the dimensions of the brush housing 128 in detail, since the cervical inspection device 110 is viewed end on. The central bore 120 is formed as a cylindrical void, and the cervical sampling brush 122 is adapted to fold or retract into a condition which is receivable within the central bore 120 fully, with the central bore 120 most preferably being dimensioned such that the cervical sampling brush 122 can be moved through the central bore 120. However, the brush housing 128 may, as illustrated, having an internal stop 147 which inhibits full removal of the brush 122 from the brush housing 128, such that it can only be removed or loaded from the second end 116. The folding or retraction means may be initiated or urged through contact with an outer rim 148 of the central bore 120 of the brush housing 128, or indeed any structure which forms or comprises part of the central bore 120. Alternatively, the cervical sampling brush could itself be provided with an actuation mechanism to cause folding. Crucially, the folding is such that the bristles of the brush 122 do not contact the walls of the brush housing 128, as will be described in more detail below.

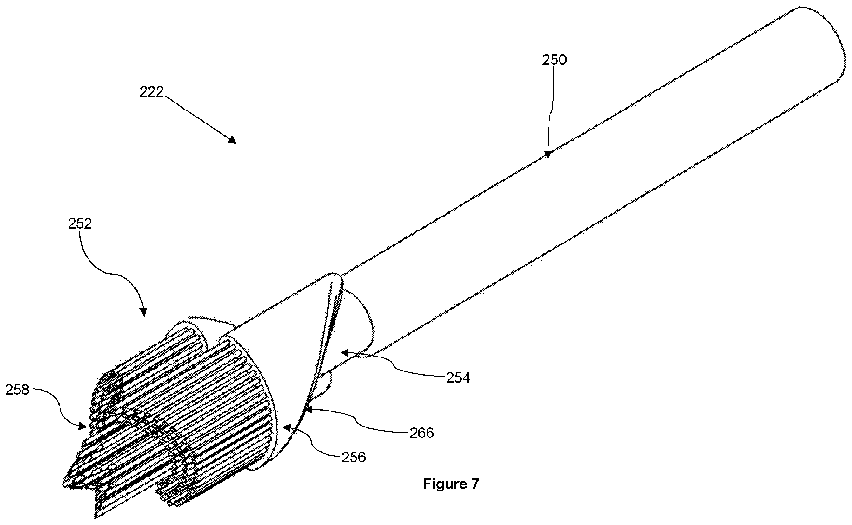

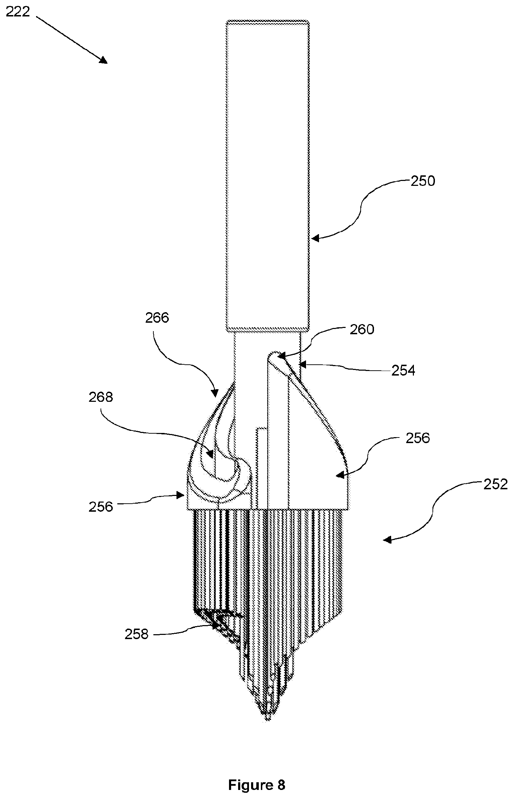

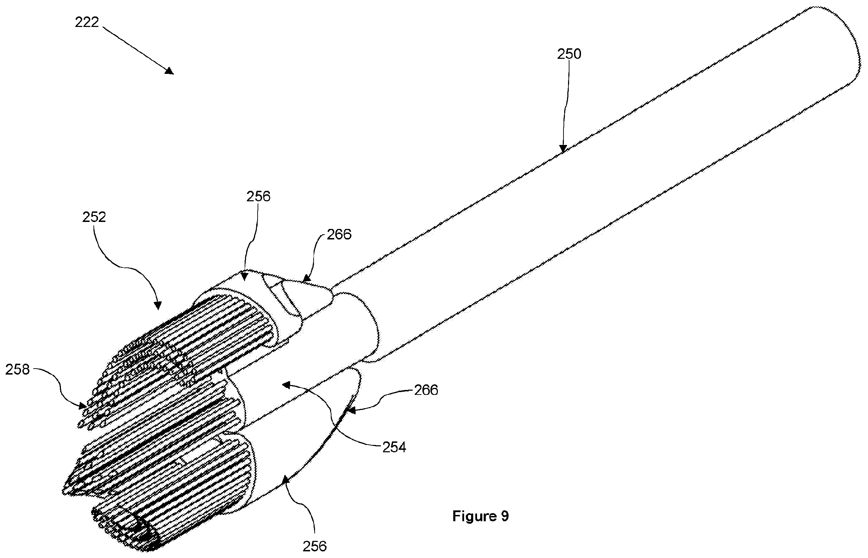

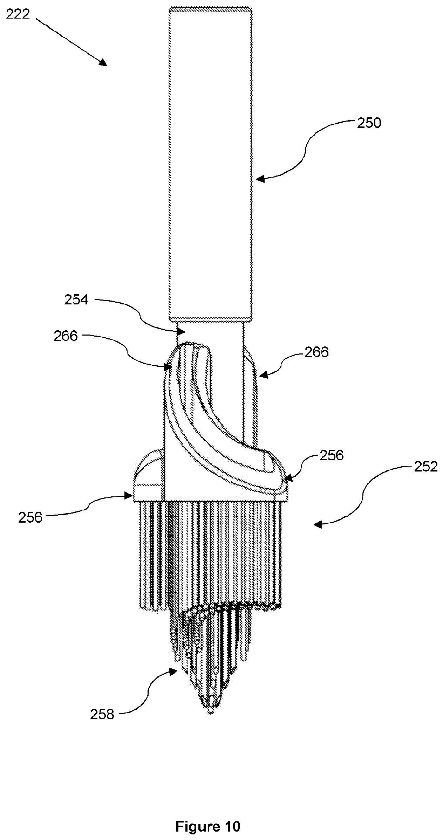

The structure of an indicative, and indeed presently preferred, embodiment of cervical sampling brush 222 is illustrated in Figures 7 to 11D. Figures 7, 8, 11A, and 11B show the cervical sampling brush 222 in a closed or retracted condition or state, and Figures 9, 10, 11C, and 11D show the cervical sampling brush 222 in an open or sampling condition or state.

The cervical sampling brush 222 has an elongate stem 250, which here is indicated as a generalised cylindrical handle, and is not necessarily depicted to scale. At one end of the elongate stem 250 is a cervical sampling head 252 which has a central hub 254 and a pair of lateral supports 256. The pair of lateral supports 256 are preferably diametrically opposed to one another for uniformity of movement. It is noted that a single lateral support could be considered, if this makes the folding process more straightforward, but having paired lateral supports 256 allows for a better matching of the shape of the cervical wall in the transformation zone.

The cervical sampling head 252 has a plurality of flexible sampling bristles 258 thereon, which are preferably designed for best capture properties of a cervical sample. This will typically involve longer bristles 258 at the central hub 254, which gradually become shorter towards the lateral edges of the lateral supports 256.

The lateral supports 256 have, in the open condition, a first lateral extent, which could be characterised as a radial extent, and a second lateral extent, in the closed condition, which is less than the first lateral extent. This is achieved by folding or moving of the lateral supports 256 towards the central hub 254.

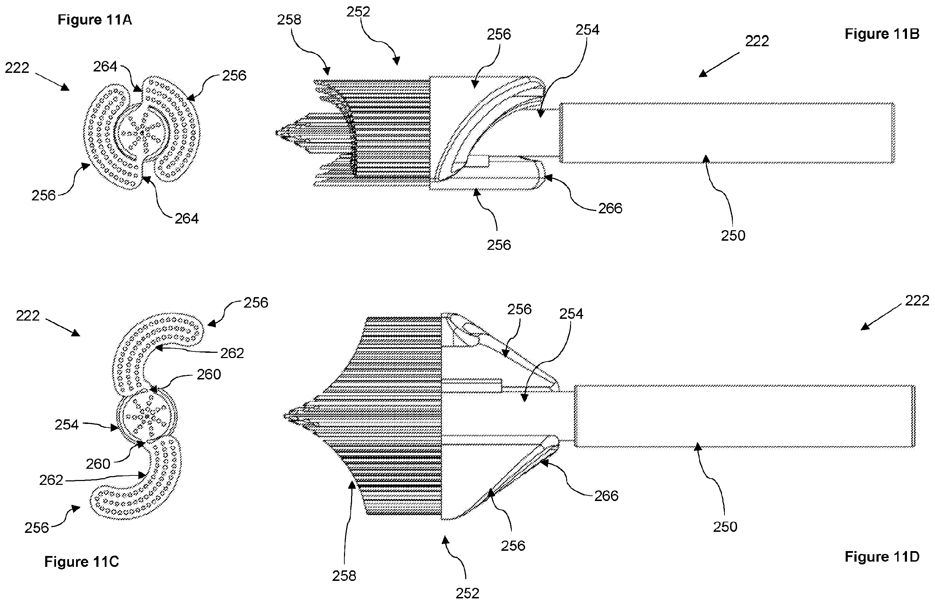

The lateral supports 256 are preferably integrally formed with the central hub 254, being connected by a hinge member 260, which may preferably be a resiliently flexible hinge. This can be best visualised in Figures 11A and 11C which show the open and closed conditions of the lateral supports 256 most clearly. In this instance, a hinge axis of the hinge members 260 is parallel with or substantially parallel with the longitudinal axis of the cervical sampling brush 222.

In the closed condition, as is the case in Figures 11A and 11B, the inner surfaces 262 of the lateral supports 256 matingly meet with an outer surface of the central hub 254, and the approximate cross-section of the cervical sampling head 252 is circular.

In the open condition, as is the case in Figures 11C and 11D, the end surfaces 264 of the lateral supports 256 matingly meet with the outer surface of the central hub 254 instead, and thus the approximate cross-section of the cervical sampling head 252 becomes sinusoidal or serpentine. The hinge axes are not centrally positioned on said end surfaces 264, thereby ensuring ease of movement of the lateral supports 256.

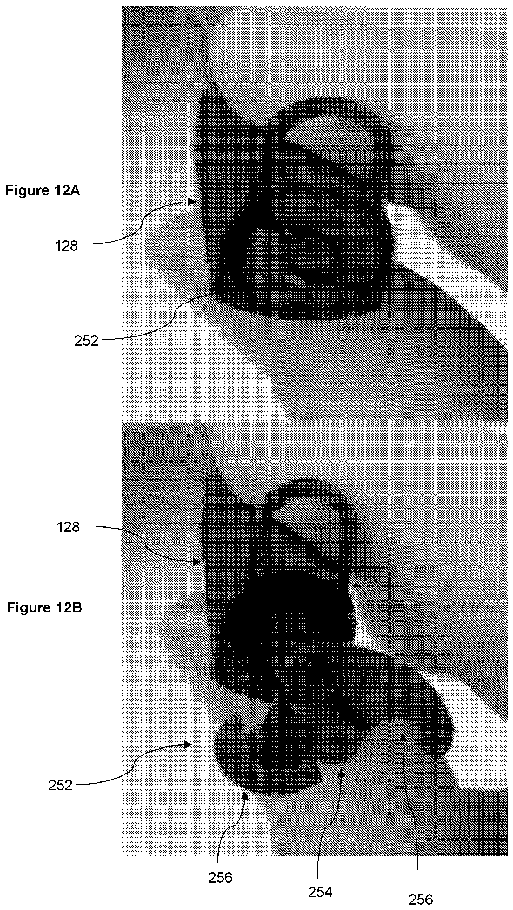

The lateral supports 256 in the present embodiment are arcuate, and more preferably rotationally symmetric, when viewed along the longitudinal axis of the cervical sampling brush 222, which allows them to curl in a spiral fashion towards the central hub 254 on folding. The bristle-distal edges 266 of the lateral supports 256 may preferably be tapered so that, upon contact with the outer rim 148 of a cervical inspection device 110, the lateral supports 256 are urged into the closed condition gradually. In the illustrated embodiment, the bristle-distal edges 266 also include a recess 268 or groove which could be used to guide the lateral supports 256 into or out of the closed condition. This process is shown in Figures 12A and 12B, with the bristles having been omitted from the cervical sampling head 252 for clarity. For the cervical sampling head 252 shown, a twisting action which accompanies the longitudinal urging of the cervical sampling brush 222 may help to encourage closure, by rotating the central hub 254 to encourage the hinges 260 to follow and thus fold up the lateral supports 254.

The bristle-distal edges 266 contact with the outer rim 148 of the brush housing 128, with the hinge member 260 resiliently moving depending on the force applied to the bristle-distal edges 266 by said outer rim 148. Thus, the motion of the cervical sampling brush 222 alone, be that by axial or axial and rotational action, causes the opening and closing of the cervical sampling head 252 and no additional control mechanism is necessary.

On retraction of the cervical sampling brush 222, the bristles 258 remain parallel with one another during the folding process, and as such, there is minimal risk of loss of sample by collision of the bristles 258 with one another or the central bore 120 of the cervical inspection device 110. There is therefore no risk of the bristles 258 becoming contaminated by the cervical inspection device 110 itself.

To use the cervical inspection device 110 with the cervical sampling brush 222, the medical practitioner inserts the cervical inspection device 110 into the patient's vaginal canal. The camera 140 is used to find and locate the cervical os and the likely transformation zone area of the cervix, once the cervical inspection device 110 has been inserted. The expansion element 130 can then be operated to urge the walls of the vaginal canal out of the way at the transformation zone, providing visual access for the camera 140 through the protection region thus formed. The cervical inspection device 110 can then be reoriented where required, based on the output of the camera 140.

Once the cervical inspection device 110 is correctly oriented, the cervical sampling brush 222 can be advanced by the medical practitioner towards the cervix. Upon leaving the central bore 120, the cervical sampling head 252 opens, with the force on the lateral supports 256 no longer being present from the brush housing 128. The resiliently flexible hinge members 260 open the lateral supports 256 into the open condition. The cervical sampling brush 222 can then be urged towards the transformation zone to collect a sample with the bristles 258.

Once sufficient sample is collected, the cervical sampling brush 222 can be retracted towards the cervical inspection device 110. Upon contact of the bristle-distal edges 266 with the outer rim 148 of the cervical inspection device 110, the taper of the bristle-distal edges 266 will gradually urge the lateral supports 256 towards the central hub 254. The cervical sampling head 252 thus closes up to itself to be receivable into the central bore 120.

Upon removal of the cervical sampling device 110 from the patient, the medical practitioner can remove the sheath 126 and brush housing 128 as one, to allow the tubular body 112 to be sanitized. The brush housing 128, containing the sampled-filled cervical sampling brush 222, can then be sent onwards for cytological analysis. Alternatively, the brush 222 can be extracted from the device 110, and the sheath 126 and brush housing 128 disposed of. Of course, it is equally feasible to extract the cervical sampling brush 222 on its own first, before washing in a cytology solution to be sent to a laboratory.

For some cytological analysis approaches, there is a requirement for the cervical sampling brush 222 to have a removable cervical sampling head 252 which can be transferred separately from the stem and/or handle of the brush in a cytology solution. This is anticipated as being feasible within all embodiments of the present invention.

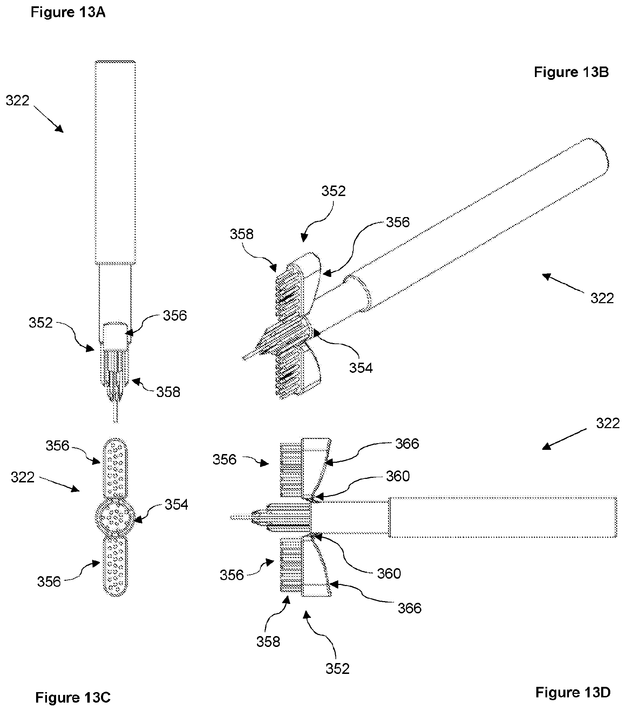

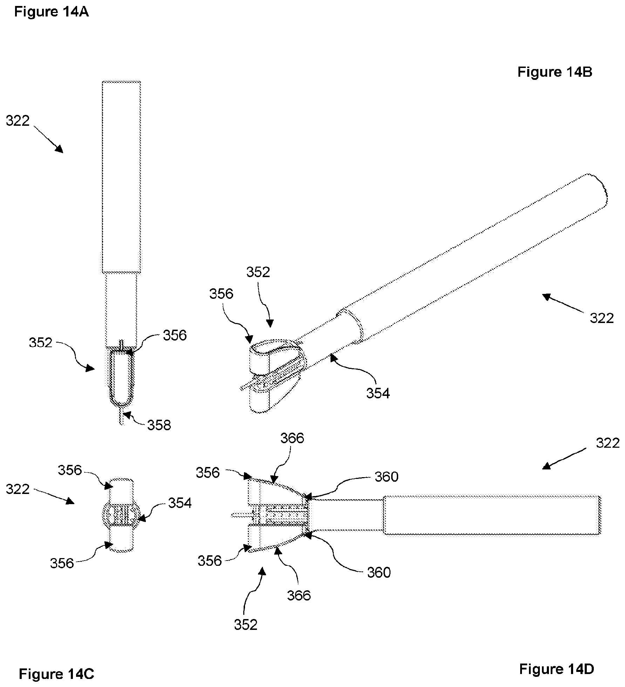

Figures 13A to 14D show a second detailed embodiment of cervical sampling brush 322. Identical or similar features of the first embodiment will be referenced using identical or similar reference numerals, and further detailed description will be omitted for brevity.

The cervical sampling head 352 comprises a pair of diametrically opposed lateral supports 356 which are respectively connected to the central hub 354 at hinge members 360. The hinge axes of the hinge members 360 are parallel to one another, and as such, the two lateral supports 356 fold towards one another so as to overlie the bristles 358 of the central hub 354. The lateral supports 356 are formed as linear supports, having tapered bristle-distal edges 366 to close in a similar manner to the first brush embodiment.

Whilst the closing of the cervical sampling head 352 in this second embodiment brings bristles 358 into contact with one another, the closing mechanism may better protect the sample following extraction of the cervical sampling brush 322 from the patient, since the bristles 358 are no longer exposed. The risk of contamination decreases, reducing the risk of needing to reperform the cervical smear test.

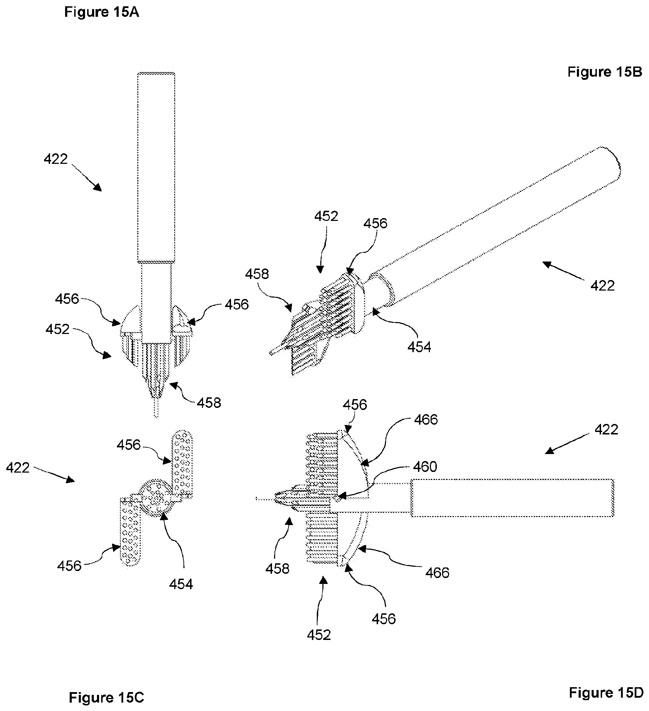

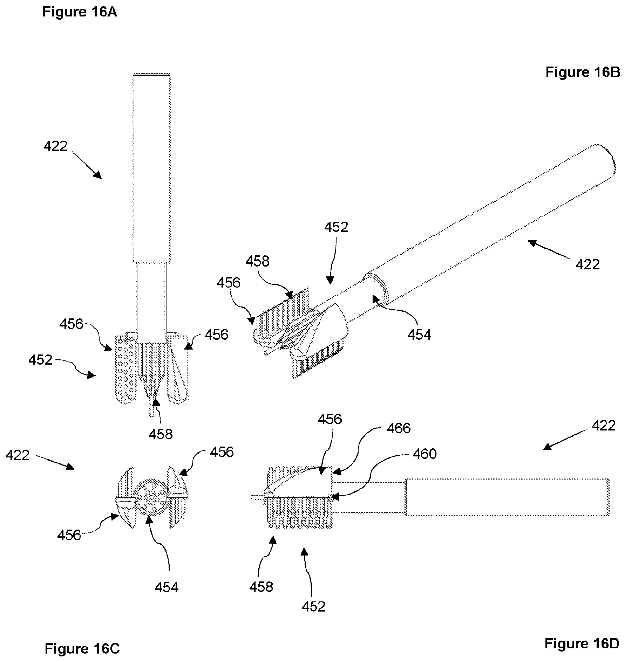

Figures 15A to 16D show a third detailed embodiment of cervical sampling brush 422. Identical or similar features of the first and second embodiments will be referenced using identical or similar reference numerals, and further detailed description will be omitted for brevity.

The cervical sampling head 452 comprises a pair of diametrically opposed lateral supports 456 which are respectively connected to the central hub 454 at hinge members 460. The hinge axes of the hinge members 460 are co-axial and perpendicular to the longitudinal axis, and as such, the two lateral supports 456 fold towards one another without contacting one another. The lateral supports 456 remain spaced apart from one another either side of the central hub 454. The lateral supports 456 are formed as linear supports, having tapered bristle-distal edges 466 to close in a similar manner to the first and second brush embodiments.

Unlike the previous two embodiments, the hinge member 460 is here not formed as a resiliently flexible hinge, but as a continuous axle which extends through the cervical sampling head 452. Whilst a resiliently flexible hinge could be considered, a specific axle is less likely to break under the increased rotational stress.

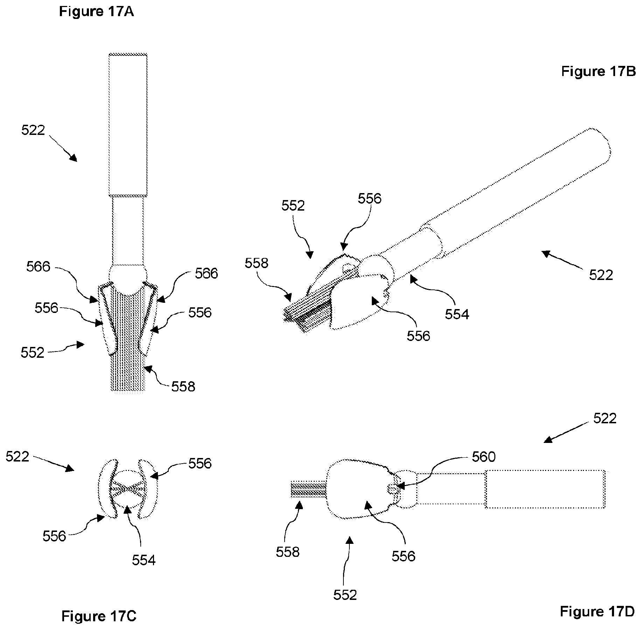

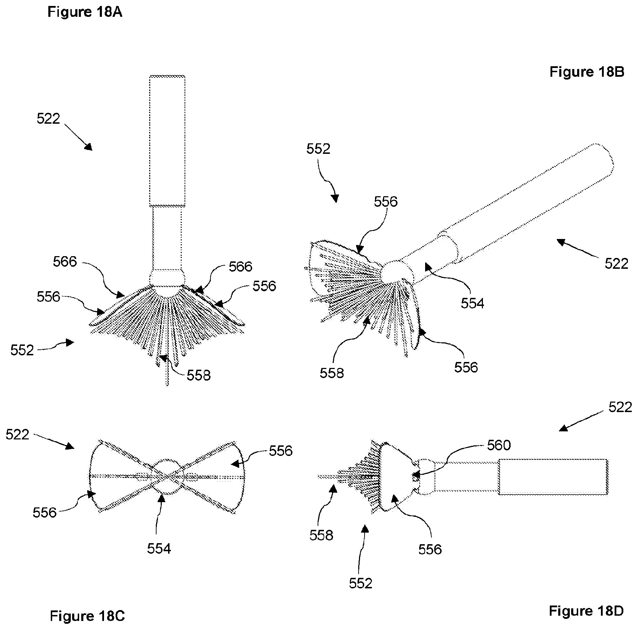

Figures 17A to 18D show a fourth detailed embodiment of cervical sampling brush 522. Identical or similar features of the first, second, and third embodiments will be referenced using identical or similar reference numerals, and further detailed description will be omitted for brevity.

The cervical sampling head 552 comprises a pair of diametrically opposed lateral supports 556 which are respectively connected to the central hub 554 at hinge members 560. The hinge axes of the hinge members 560 are parallel to one another, and as such, the two lateral supports 556 fold towards one another so as to overlie the bristles 558 of the central hub 554. The lateral supports 556 are formed as fan-shaped supports which may allow novel bristle 558 configurations to be used which can better capture a wider area beyond the transformation zone during the cervical smear test.

Again, whilst the closing of the cervical sampling head 552 in this fourth embodiment brings bristles 558 into contact with one another, the closing mechanism may better protect the sample following extraction of the cervical sampling brush 522 from the patient, since the bristles 558 are no longer exposed. The risk of contamination decreases, reducing the risk of needing to reperform the cervical smear test.

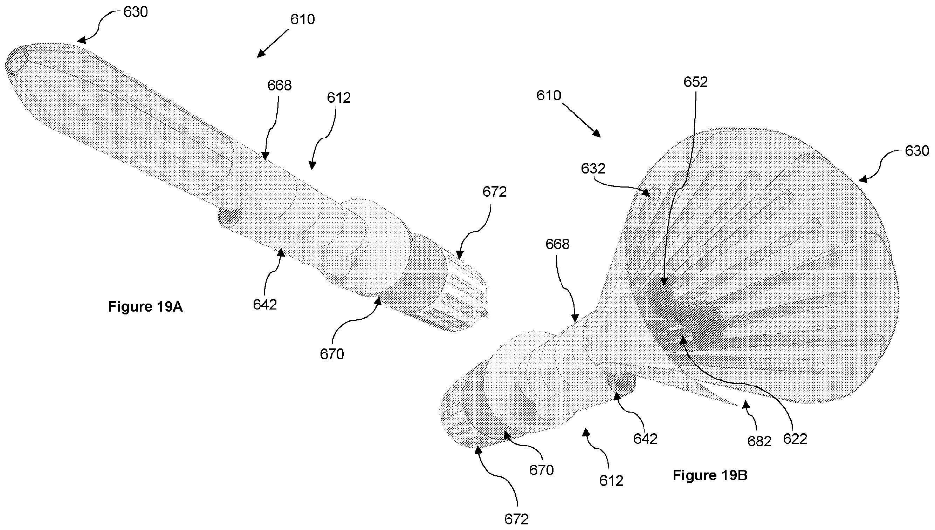

Figures 19A to 20B show a further embodiment of a cervical inspection device, referenced globally at 610. Identical or similar features of the previous embodiments will be referenced using identical or similar reference numerals, and further detailed description will be omitted for brevity.

The cervical inspection device 610 has a tubular body 612 formed in two parts, having the central bore 620 running through the whole tubular body 612. There is a leading portion 668 within which is located a moveable brush housing 628, and a trailing portion 670 which includes the control means for the expansion element 630. It may be possible to provide the leading portion 668 as a disposable unit, which can be jettisoned after use and replaced with a sterile new leading portion 668 for the next patient.

The control means is here formed as a rotary member 672 formed on the tubular body 612 via which an internal screw thread 674 in the tubular body 612 can be driven to advance or retract the brush housing 628. The cervical sampling brush 622 can, however, be advanced or retracted by the operator separately to the movement of the brush housing 628.

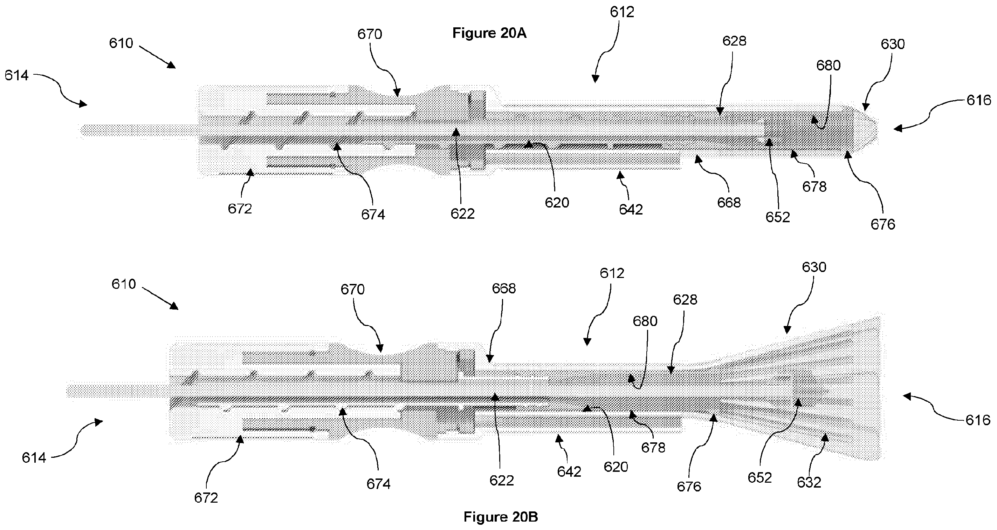

The expansion element 630 is formed as a hooded portion which splays into or substantially into the shape of a cone when activated. There is a plurality of tabs 632 positioned at the second end 616 of the cervical inspection device 610 which are splayed when there is an interference fit formed with an end of the brush housing 628 in the expanded condition. This occurs when the brush housing 628 has been withdrawn towards the first end 614 of the tubular body 612. An engorged lip 676 of the brush housing 628, which overhangs a primary outer wall 678 thereof, causes the interference expansion to occur, splaying the tabs 632 outwardly, as best seen in Figures 19B and 20B. The hooded portion is here shown as being a fluted paper hood, and a fluted structure may prove straightforward to allow for expansion and retraction.

Before use, the cervical sampling brush 622 is completely concealed within the brush housing 628, which is advanced towards the second end 616 of the tubular body 612. The cervical sampling brush 622 is kept in an unfurled condition by the internal walls 680 of the brush housing 628. As the brush housing 628 is withdrawn, the cervical sampling brush 622 is uncovered, and it unfurls. A cervical sampling brush 622 akin to that of Figures 11A to 11D is illustrated. The positioning of the cervical sampling brush 622 is such that, splaying of the tabs 632 occurs before complete release of the cervical sampling head 652, and thus the expansion element 630 is splayed sufficiently before unfurling of the cervical sampling brush 622, so that the cervical sampling head 652 never contacts the expansion element 630. The expansion element 630, in the expanded condition, thus forms a protective region around the cervical sampling brush 622, so that it can in-use be advanced towards the cervix without the cervical sampling brush 622 ever needing to be exposed to the vaginal canal.

Targeted sampling of the transformation zone of the cervix can thus be achieved without contamination, with the camera being positioned within the radial extent of the expansion element 630 once expanded. The camera, indicated here by the presence of a camera receiving area 642, allows for inspection of the transformation zone through the protective region. The camera could itself be positioned within the protective region formed by the expansion element 630, or there could be a viewing corridor 682 in the protective region, as illustrated.

It will be appreciated that, although the cervical inspection device has been described for use with a cervical sampling brush, the device has a wide utility in cervical sampling contexts. In particular, there are other cervical inspection tools which are available which could be inserted through the central bore of the device, such as a sprayer suitable for spraying acetic acid onto the transformation zone in order to improve the identification of cellular markers. The brush housing which forms the central bore as herebefore described thus could readily be any cervical inspection tool housing, which could be disposed after use.

It is therefore possible to provide a generalised method of controlling a cervical inspection device for performing a cervical smear test, the method comprising the steps of: providing a cervical inspection device as previously described; pushing the cervical sampling brush through the central bore of the tubular body to permit opening of the cervical sampling head into the sampling condition; and pulling the cervical sampling brush into the central bore of the tubular body to urge the lateral supports of the cervical sampling brush to fold towards the central hub upon contact of the lateral supports with the tubular body. Regardless of which brush type is used, the folding mechanism can be achieved, and a less stressful cervical smear test completed for the patient.

The presence of a camera and the engagement elements to allow for improved inspection of the cervical transformation zone is a significant advance over the state of the art, reducing the risk of discomfort to the patient, particularly those patients with retroverted uteruses. Irrespective of the brush or swab used, the present invention therefore also provides for a cervical inspection device for performing a cervical smear test, or other cervical inspection techniques, the device comprising: a tubular body having a first end which is engagable by an operator and a second end which is insertable into a patient's vaginal canal, the tubular body having a central bore which extends along its longitudinal axis; a cervical brush or swab insertable through the central bore of the tubular body; an expansion element at or adjacent to the second end of the tubular body; control means operable from the first end of the tubular body for selectably expanding and/or contracting the expansion element; and a camera at or adjacent to second end of the tubular body and which is positioned radially outwardly of the central bore. This achieves the aim of reduced-pain cervical smear testing, eliminating the need for a speculum.

Overall, the present invention provides a means of improving the overall experience of the cervical smear test for women, hopefully improving uptake, and reducing the risk of sample contamination.

The words 'comprises/comprising' and the words 'having/including' when used herein with reference to the present invention are used to specify the presence of stated features, integers, steps, or components, but do not preclude the presence or addition of one or more other features, integers, steps, components, or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

The embodiments described above are provided by way of examples only, and various other modifications will be apparent to persons skilled in the field without departing from the scope of the invention as defined in the claims.

## Claims

1. A cervical inspection device (10; 110; 610) for performing a cervical smear test, the device (10; 110; 610) comprising:
a tubular body (12; 112; 612) having a first end (14; 114; 614) which is engagable by an operator and a second end (16; 116; 616) which is insertable into a patient's vaginal canal, the tubular body (12; 112; 612) having a central bore (20; 120; 620) which extends along its longitudinal axis for receiving a cervical sampling brush (22; 222; 322; 422; 522; 622) therethrough;
an expansion element (30; 630) at the second end (16; 116; 616) of the tubular body (12; 112; 612); and
control means operable from the first end (14; 114; 614) of the tubular body (12; 112; 612) for selectably expanding and/or contracting the expansion element (30); and
the expansion element (30; 630) forming a circumferentially-bound protective region beyond the central bore (20; 120; 620) in an expanded condition thereof for the said cervical sampling brush (22; 222; 322; 422; 522; 622) to move through during a cervical smear test; and
a camera (40) at or adjacent to the second end of the tubular body (12; 112; 612) and positioned radially inwardly of the expansion element (30; 630); and
the expansion element (30; 630) comprises a flexible hood (31), the control means comprising a plurality of tabs (32; 132; 632) for splaying the flexible hood (31) into an open condition,
**characterized by** the plurality of tabs (32; 132; 632) each being connected to the second end (16 116; 616) of the tubular body (12; 112; 612) by a hinge and the camera being positioned radially outwardly of and spaced apart from the central bore (20; 120; 620).

2. A cervical inspection device (10; 110; 612) as claimed in claim 1, further comprising a camera receiving area (42; 642) at or adjacent to an end of the channel which is laterally spaced apart from the central bore (20; 120; 620) and within a maximum lateral extent of the expansion element (30; 630) in the expanded condition.

3. A cervical inspection device (10; 110; 610) as claimed in claim 2, wherein the camera (40) is angled towards an axis of the central bore (20; 120; 620) for monitoring the cervical sampling brush (22; 222; 322; 422; 522; 622) in use.

4. A cervical inspection device (10; 110; 610) as claimed in claim 2 or claim 3, further comprising an illumination element associated with the camera (40).

5. A cervical inspection device (10; 110; 610) as claimed in any one of the preceding claims, wherein the expansion element (30; 630) has a conical, frusto-conical, substantially conical, or substantially frusto-conical shape in the expanded condition.

6. A cervical inspection device (10; 110; 610) as claimed in any one of the preceding claims, wherein a distal rim of the expansion element (30; 630) in-use forms a cervical-proximal edge of the protection zone to form an enclosure with the cervix.

7. A cervical inspection device (10; 110; 610) as claimed in any one of the preceding claims, further comprising a sheath (26) which is receivable over the tubular body (12; 112; 612).

8. A cervical inspection device (10; 110; 610) as claimed in any one of the preceding claims, wherein the control means engages with the expansion element (30; 630) in an interference fit to urge the expansion element (30; 630) into the expanded condition.

9. A cervical inspection device (10; 110; 610) as claimed in any one of the preceding claims, wherein the expansion element (30; 630) is dimensioned to form the circumferentially-bound protective region in-use around a cervix to be inspected in the expanded condition.

10. A cervical inspection device (10; 110; 610) as claimed in any one of the preceding claims, wherein the hinges are located at or adjacent to the camera (40) at the second end of the tubular body (12; 112; 612).

11. A cervical inspection device (10; 110; 610) as claimed in any one of the preceding claims, further comprising a cervical sampling brush (22; 222; 322; 422; 522; 622) comprising:
an elongate stem (250); and
a cervical sampling head (252; 352; 452; 552; 652) having a central hub (254; 354; 454; 554) and a lateral support (256; 356; 456; 556), the cervical sampling head (252; 352; 452; 452; 652) having a plurality of flexible sampling bristles (258; 358; 458; 558) thereon;
wherein in a sampling condition, the lateral support (256; 356; 456; 556) has a first lateral extent relative to the elongate stem (250), and in a retraction condition, the lateral support (256; 356; 456; 556) is movable or foldable towards an axis of the elongate stem (250) to adopt a second lateral extent which is less than the first lateral extent, so as to be receivable into the tubular body (12; 112; 612) without the plurality of flexible sampling bristles (258; 358; 458; 558) contacting the tubular body (12; 112; 612) or the expansion element (30; 630).

12. A cervical inspection device (10; 110; 610) as claimed in claim 11, wherein the or each lateral support (256; 356; 456; 556) has a tapered bristle-distal edge (266; 366; 466; 566) to urge the or each lateral support (256; 356; 456; 556) into the retracted condition upon application of an axial force.

13. A cervical inspection device (10; 110; 610) as claimed in claim 11 or claim 12, wherein the operation of the control means is independent of the movement of the cervical sampling brush (22; 222; 322; 422; 522; 622).

14. A method of controlling a cervical inspection device (10; 110; 610) for performing a cervical smear test, the method comprising the steps of:
providing a cervical inspection device (10; 110; 610) as claimed in any one of the preceding claims, the expansion element (30; 630) being in a contracted condition; and
using the control means, expanding the expansion element (30; 630) into the expanded condition to a protective region beyond the central bore (20; 120; 620) through which a cervical sampling brush (22; 222; 322; 422; 522; 622) can pass.

15. A method of controlling a cervical inspection device (10; 110; 610) for performing a cervical smear test, the method comprising the steps of:
providing a cervical inspection device (10; 110; 610) as claimed in any one of claims 11 to 13;
pushing the cervical sampling brush (22; 222; 322; 422; 522; 622) through the central bore (20; 120; 620) of the tubular body (12; 112; 612) to permit opening of the cervical sampling head (252; 352; 452; 552; 652) into the sampling condition; and
pulling the cervical sampling brush (22; 222; 322; 422; 522; 622) into the central bore (20; 120; 620) of the tubular body (12; 112; 612) to urge the lateral supports (256; 356; 456; 556) of the cervical sampling brush (22; 222; 322; 422; 552; 652) to fold towards the central hub (254; 354; 454; 554) upon contact of the lateral supports (256; 356; 456; 556) with the tubular body (12; 112; 612).

## Patentansprüche

1. Vorrichtung zur zervikalen Inspektion (10; 110; 610) zur Durchführung eines zervikalen Abstrichtests, wobei die Vorrichtung (10; 110; 610) umfasst:
einen röhrenförmigen Körper (12; 112; 612), aufweisend ein erstes Ende (14; 114; 614), das durch einen Bediener einsetzbar ist, und ein zweites Ende (16; 116; 616), das in einen Vaginalkanal einer Patientin eingreifbar ist, wobei der röhrenförmige Körper (12; 112; 612) eine zentrale Bohrung (20; 120; 620) aufweist, die sich entlang seiner Längsachse zum Aufnehmen einer Bürste zur zervikalen Probenahme (22; 222; 322; 422; 522; 622) erstreckt;
ein Ausdehnungselement (30; 630) an dem zweiten Ende (16; 116; 616) des röhrenförmigen Körpers (12; 112; 612); und
ein Steuermittel, das von dem ersten Ende (14; 114; 614) des röhrenförmigen Körpers (12; 112; 612) bedienbar ist, zum wählbaren Ausdehnen und/oder Zusammenziehen des Ausdehnungselements (30); und
wobei das Ausdehnungselement (30; 630) in einem ausgedehnten Zustand davon eine umfänglich abgegrenzte Schutzregion über die zentrale Bohrung (20; 120; 620) hinaus bildet, durch die sich die besagte Bürste zur zervikalen Probenahme (22; 222; 322; 422; 522; 622) während eines zervikalen Abstrichtests bewegen kann; und
eine Kamera (40) an oder angrenzend an das zweite Ende des röhrenförmigen Körpers (12; 112; 612) und von dem Ausdehnungselement (30; 630) radial nach innen positioniert; und wobei das Ausdehnungselement (30; 630) eine flexible Haube (31) bildet, wobei das Steuermittel eine Vielzahl von Schlaufen (32; 132; 632) zum Spreizen der flexiblen Haube (31) in einen offenen Zustand umfasst;
**dadurch gekennzeichnet, dass** die Vielzahl von Schlaufen (32; 132; 632) jeweils durch ein Gelenk mit dem zweiten Ende (16; 116; 616) des röhrenförmigen Körpers (12; 112; 612) verbunden ist und die Kamera von der zentralen Bohrung (20; 120; 620) radial nach außen und davon beabstandet positioniert ist.

2. Vorrichtung zur zervikalen Inspektion (10; 110; 612) nach Anspruch 1, ferner umfassend einen Kameraaufnahmebereich (42; 642) an oder angrenzend an ein Ende des Kanals, der lateral von der zentralen Bohrung (20; 120; 620) beabstandet und innerhalb einer maximalen lateralen Erstreckung des Ausdehnungselements (30; 630) in dem ausgedehnten Zustand ist.

3. Vorrichtung zur zervikalen Inspektion (10; 110; 610) nach Anspruch 2, wobei die Kamera (40) zu einer Achse der zentralen Bohrung (20; 120; 620) hin zur Überwachung der Bürste zur zervikalen Probenahme (22; 222; 322; 422; 522; 622) abgewinkelt sind.

4. Vorrichtung zur zervikalen Inspektion (10; 110; 610) nach Anspruch 2 oder Anspruch 3, ferner umfassend ein Beleuchtungselement, das mit der Kamera (40) assoziiert ist.

5. Vorrichtung zur zervikalen Inspektion (10; 110; 610) nach einem der vorhergehenden Ansprüche, wobei das Ausdehnungselement (30; 630) in dem ausgedehnten Zustand eine kegelförmige, kegelstumpfförmige, im Wesentlichen kegelförmige oder im Wesentlichen kegelstumpfförmige Form aufweist.

6. Vorrichtung zur zervikalen Inspektion (10; 110; 610) nach einem der vorhergehenden Ansprüche, wobei ein distaler Rand des Ausdehnungselements (30; 630) im Gebrauch eine zervikal-proximale Kante der Schutzzone bildet, um ein Umfassung mit der Zervix zu bilden.

7. Vorrichtung zur zervikalen Inspektion (10; 110; 610) nach einem der vorhergehenden Ansprüche, ferner umfassend eine Schleuse (26), die über dem röhrenförmigen Körper (12; 112; 612) aufnehmbar ist.

8. Vorrichtung zur zervikalen Inspektion (10; 110; 610) nach einem der vorhergehenden Ansprüche, wobei das Steuermittel das Ausdehnungselement (30; 630) in einer Presspassung in Eingriff nimmt, um das Ausdehnungselement (30; 630) in den ausgedehnten Zustand zu treiben.

9. Vorrichtung zur zervikalen Inspektion (10; 110; 610) nach einem der vorhergehenden Ansprüche, wobei das Ausdehnungselement (30; 630) dimensioniert ist, um in dem ausgedehnten Zustand im Gebrauch die umfänglich abgegrenzte Schutzregion um eine zu inspizierende Zervix zu bilden.

10. Vorrichtung zur zervikalen Inspektion (10; 110; 610) nach einem der vorhergehenden Ansprüche, wobei sich die Gelenke an oder angrenzend an die Kamera (40) an dem zweiten Ende des röhrenförmigen Körpers (12; 112; 612) befinden.

11. Vorrichtung zur zervikalen Inspektion (10; 110; 610) nach einem der vorhergehenden Ansprüche, ferner umfassend eine Bürste zur zervikalen Probenahme (22; 222; 322; 422; 522; 622), umfassend:
einen länglichen Schaft (250); und
einen Kopf zur zervikalen Probenahme (252; 352; 452; 552; 652), aufweisend eine zentrale Nabe (254; 354; 454; 554) und einen lateralen Träger (256; 356; 456; 556), wobei der Kopf zur zervikalen Probenahme (252; 352; 452; 452; 652) eine Vielzahl von flexiblen Probenahmeborsten (258; 358; 458; 558) darauf aufweist;
wobei der laterale Träger (256; 356; 456; 556) in einem Probenahmezustand eine erste laterale Erstreckung in Bezug auf den länglichen Schaft (250) aufweist und der laterale Träger (256; 356; 456; 556) in einem Einfahrzustand zu einer Achse des länglichen Schafts (250) hin bewegbar oder klappbar ist, um eine zweite laterale Erstreckung anzunehmen, die kleiner als die erste laterale Erstreckung ist, um in dem röhrenförmigen Körper (12; 112; 612) aufnehmbar ist, ohne dass die Vielzahl von flexiblen Probenahmeborsten (258; 358; 458; 558) den röhrenförmigen Körper (12; 112; 612) oder das Ausdehnungselement (30; 630) berührt.

12. Vorrichtung zur zervikalen Inspektion (10; 110; 610) nach Anspruch 11, wobei der oder jeder laterale Träger (256; 356; 456; 556) eine verjüngte borstendistale Kante (266; 366; 466; 566) aufweist, um den oder jeden lateralen Träger (256; 356; 456; 556) bei Ausübung einer Axialkraft in den eingefahrenen Zustand zu treiben.

13. Vorrichtung zur zervikalen Inspektion (10; 110; 610) nach Anspruch 11 oder Anspruch 12, wobei die Bedienung des Steuermittels von der Bewegung der Bürste zur zervikalen Probenahme (22; 222; 322; 422; 522; 622) unabhängig ist.

14. Verfahren zur Steuerung einer Vorrichtung zur zervikalen Inspektion (10; 110; 610) zur Durchführung eines zervikalen Abstrichtests, wobei das Verfahren die Schritte umfasst:
Bereitstellen einer Vorrichtung zur zervikalen Inspektion (10; 110; 610) nach einem der vorhergehenden Ansprüche, wobei das Ausdehnungselement (30; 630) in einem zusammengezogenen Zustand ist; und
unter Verwendung des Steuermittels Ausdehnen des Ausdehnungselements (30; 630) in den ausgedehnten Zustand zu einer Schutzregion über die zentrale Bohrung (20; 120; 620) hinaus, durch die eine Bürste zur zervikalen Probenahme (22; 222; 322; 422; 522; 622) hindurchtreten kann.

15. Verfahren zur Steuerung einer Vorrichtung zur zervikalen Inspektion (10; 110; 610) zur Durchführung eines zervikalen Abstrichtests, wobei das Verfahren die Schritte umfasst:
Bereitstellen einer Vorrichtung zur zervikalen Inspektion (10; 110; 610) nach einem der Ansprüche 11 bis 13;
Schieben der Bürste zur zervikalen Probenahme (22; 222; 322; 422; 522; 622) durch die zentrale Bohrung (20; 120; 620) des röhrenförmigen Körpers (12; 112; 612), um eine Öffnung des Kopfs zur zervikalen Probenahme (252; 352; 452; 552; 652) in den Probenahmezustand zu ermöglichen; und
Ziehen der Bürste zur zervikalen Probenahme (22; 222; 322; 422; 522; 622) in die zentrale Bohrung (20; 120; 620) des röhrenförmigen Körpers (12; 112; 612), um die lateralen Träger (256; 356; 456; 556) der Bürste zur zervikalen Probenahme (22; 222; 322; 422; 522; 622) zu treiben, um sich bei Berührung der lateralen Träger (256; 356; 456; 556) mit dem röhrenförmigen Körper (12; 112; 612) zu der zentralen Nabe (254; 354; 454; 554) hin zu klappen.

## Revendications

1. Un dispositif d'inspection cervicale (10 ; 110 ; 610) destiné à réaliser un prélèvement cervical, ledit dispositif
(10 ; 110 ; 610) comprenant :
un corps tubulaire (12 ; 112 ; 612) présentant une première extrémité (14 ; 114 ; 614) pouvant être engagée par un opérateur et une seconde extrémité (16 ; 116 ; 616) pouvant être introduite dans le canal vaginal d'une patiente, le corps tubulaire (12 ; 112 ; 612) présentant un alésage central (20 ; 120 ; 620) s'étendant le long de son axe longitudinal et destiné à recevoir un écouvillon de prélèvement cervical (22 ; 222 ; 322 ; 422 ; 522 ;
622) à travers celui-ci ;
un élément d'expansion (30 ; 630) disposé au niveau de la seconde extrémité (16 ; 116 ; 616) du corps tubulaire (12 ; 112 ; 612) ; et
des moyens de commande pouvant être actionnés depuis la première extrémité (14 ; 114 ; 614) du corps tubulaire (12 ; 112 ; 612) afin de provoquer sélectivement l'expansion et/ou la contraction de l'élément d'expansion (30 ; 630) ; ledit élément d'expansion (30 ; 630) formant, lorsqu'il est en position déployée, une zone de protection délimitée de manière circonférentielle au-delà de l'alésage central (20 ; 120 ; 620), à travers laquelle ledit écouvillon de prélèvement cervical (22 ; 222 ; 322 ; 422 ; 522 ; 622) peut se déplacer lors d'un prélèvement cervical ; et
une caméra (40) disposée au niveau de la seconde extrémité du corps tubulaire (12 ; 112 ; 612) ou à proximité de celle-ci,
et positionnée de manière radiale vers l'intérieur par rapport à l'élément
d'expansion (30 ; 630) ; et
l'élément d'expansion (30 ; 630) comprend une coiffe flexible (31), les moyens de commande comprenant une pluralité de languettes (32 ; 132 ; 632) destinées à déployer la coiffe flexible (31) pour l'amener dans une configuration ouverte et **caractérisé en ce que**
chacune de la pluralité de languettes (32 ; 132 ; 632) est reliée à la seconde extrémité (16 116 ; 616) du corps tubulaire (12 ; 112 ; 612) par une charnière, et la caméra étant positionnée de manière radiale vers l'extérieur par rapport à l'alésage central (20 ; 120 ; 620) et espacée de celui-ci.

2. Un dispositif d'inspection cervicale (10 ; 110 ; 610) selon la revendication 1, comprenant en outre une zone de réception de caméra (42 ; 642) située au niveau d'une extrémité du canal ou à proximité de celle-ci, ledit canal étant espacé latéralement de l'alésage central (20 ; 120 ; 620), et situé dans les limites de l'étendue latérale maximale de l'élément d'expansion (30 ; 630) lorsqu'il est en position déployée.

3. Un dispositif d'inspection cervicale (10 ; 110 ; 610) selon la revendication 2, dans lequel la caméra (40) est inclinée vers un axe
de l'alésage central (20 ; 120 ; 620) afin de surveiller l'écouvillon de prélèvement cervical (22 ; 222 ; 322 ; 422 ; 522 ; 622) en cours d'utilisation.

4. Un dispositif d'inspection cervicale (10 ; 110 ; 610) selon la revendication 2 ou la revendication 3, comprenant en outre un élément d'éclairage associé à la caméra (40).

5. Un dispositif d'inspection cervicale (10; 110 ; 610) selon l'une quelconque des revendications précédentes, dans lequel l'élément d'expansion (30 ; 630) présente, lorsqu'il est en position déployée, une forme conique, tronconique, sensiblement conique ou sensiblement tronconique.

6. Un dispositif d'inspection cervicale (10; 110 ; 610) selon l'une quelconque des revendications précédentes, dans lequel, en cours d'utilisation, un bord distal de l'élément d'expansion (30 ; 630) forme un bord proximal par rapport au col de l'utérus de la zone de protection, de manière à former une enceinte avec le col de l'utérus.

7. Un dispositif d'inspection cervicale (10 ; 110 ; 610) tel que revendiqué dans l'une quelconque des revendications précédentes, comprenant en outre une gaine (26) qui peut être reçue sur le corps tubulaire (12 ; 112 ; 612).

8. Un dispositif d'inspection cervicale (10; 110 ; 610) selon l'une quelconque des revendications précédentes, dans lequel les moyens de commande s'engagent avec l'élément d'expansion (30 ; 630) par ajustement avec interférence afin de solliciter l'élément d'expansion (30 ; 630) pour l'amener dans sa configuration déployée.

9. Un dispositif d'inspection cervicale (10; 110 ; 610) selon l'une quelconque des revendications précédentes, dans lequel l'élément d'expansion (30 ; 630) est dimensionné de manière à former, en cours d'utilisation et lorsqu'il est en configuration déployée, la zone de protection délimitée de manière circonférentielle autour d'un col de l'utérus à inspecter.

10. Un dispositif d'inspection cervicale (10 ; 110 ; 610) selon l'une quelconque des revendications précédentes, dans lequel les charnières sont situées au niveau ou à proximité de la caméra (40), à la seconde extrémité du corps tubulaire (12 ; 112 ; 612).

11. Un dispositif d'inspection cervicale (10 ; 110 ; 610) selon l'une quelconque des revendications précédentes, comprenant en outre un écouvillon de prélèvement cervical (22 ; 222 ; 322 ; 422 ; 522 ; 622) comprenant :
une tige allongée (250) ;
et une tête de prélèvement cervical (252 ; 352 ; 452 ; 552 ; 652) présentant un moyeu central (254 ; 354 ; 454 ; 554) et un support latéral (256 ; 356 ; 456 ; 556), la tête de prélèvement cervical (252 ; 352 ;
452 ; 552 ; 652) présentant une pluralité de soies de prélèvement flexibles (258 ; 358 ; 458 ; 558) ; et
dans laquelle, dans une configuration de prélèvement, le support latéral (256 ; 356 ; 456 ; 556) présente une première étendue latérale par rapport à la tige allongée (250), et, dans une configuration de rétraction, le support latéral (256 ; 356 ; 456 ; 556) est mobile ou pliable vers un axe de la tige allongée (250) pour adopter une seconde étendue latérale inférieure à la première étendue latérale, de manière à pouvoir être introduite dans le corps tubulaire (12 ; 112 ; 612) sans que la pluralité de soies de prélèvement flexibles (258 ; 358 ; 458 ; 558) n'entre en contact avec le corps tubulaire (12 ; 112 ; 612) ou avec l'élément d'expansion (30 ; 630).

12. Un dispositif d'inspection cervicale (10 ; 110 ; 610) selon la revendication 11, dans lequel ledit support latéral ou chacun des supports latéraux (256 ; 356 ; 456 ; 556) présente un bord effilé distal par rapport aux soies (266 ; 366 ; 466 ; 566), destiné à solliciter ledit support latéral ou chacun des supports latéraux (256 ; 356 ; 456 ; 556) pour l'amener dans la configuration de rétraction lorsqu'une force axiale est appliquée.

13. Un dispositif d'inspection cervicale (10 ; 110 ; 610) selon la revendication 11 ou la revendication 12, dans lequel le fonctionnement des moyens de commande est indépendant du déplacement de l'écouvillon de prélèvement cervical (22 ; 222 ; 322 ; 422 ; 522 ; 622).

14. Un procédé de commande d'un dispositif d'inspection cervicale (10 ; 110 ; 610) destiné à réaliser un prélèvement cervical, le procédé comprenant les étapes consistant à :
prévoir un dispositif d'inspection cervicale (10 ; 110 ; 610) selon l'une quelconque des revendications précédentes, l'élément d'expansion (30 ; 630) étant dans une configuration rétractée ; et
en utilisant les moyens de commande, déployer l'élément d'expansion (30 ; 630) jusqu'à sa configuration déployée, de manière à former une zone de protection au-delà de l'alésage central (20 ; 120 ; 620), à travers laquelle un écouvillon de prélèvement cervical (22 ; 222 ; 322 ; 422 ; 522 ; 622) peut passer.

15. Un procédé de commande d'un dispositif d'inspection cervicale (10 ; 110 ; 610) destiné à réaliser un prélèvement cervical, le procédé comprenant les étapes consistant à :
prévoir un dispositif d'inspection cervicale (10 ; 110 ; 610) selon l'une quelconque des revendications 11 à 13 ;
faire avancer l'écouvillon de prélèvement cervical (22 ; 222 ; 322 ; 422 ; 522 ; 622) à travers l'alésage central (20 ; 120 ; 620) du corps tubulaire (12 ; 112 ; 612) afin de permettre l'ouverture de la tête de prélèvement cervical (252 ; 352 ; 452 ; 552 ; 652) pour l'amener dans la configuration de prélèvement ; et
tirer l'écouvillon de prélèvement cervical (22 ; 222 ; 322 ; 422 ; 522 ; 622) dans l'alésage central (20 ; 120 ; 620) du corps tubulaire (12 ; 112 ; 612) afin de solliciter les supports latéraux (256 ; 356 ; 456 ; 556) de l'écouvillon de prélèvement cervical (22 ; 222 ; 322 ; 422 ; 522 ; 652) pour les faire se replier vers le moyeu central (254 ; 354 ; 454 ; 554) lors du contact des supports latéraux (256 ; 356 ; 456 ;
556) avec le corps tubulaire (12 ; 112 ; 612).
